# EUROPEAN PATENT APPLICATION

(11) **EP 3 936 037 A1**
(43) Date of publication of application: **12.01.2022**
(21) Application number: 20184897.5
(22) Date of filing: 09.07.2020
(51) Int. Cl.: A61B 5/024, A61B 5/026, A61B 5/1455, A61B 5/00, G02B 6/08

(54) **OPTICAL LIGHT GUIDE FOR OPTICAL SENSOR**

(71) Applicant: Polar Electro Oy, 90440 Kempele (FI)
(72) Inventor: Korkala, Seppo, 90440 KEMPELE (FI)
(74) Representative: Kolster Oy Ab

(57) **Abstract**

The present document discloses a solution for optical biometric measurements. According to an aspect, a sensor device comprises: a sensor head configured to face a skin of a human body, the sensor head comprising at least one optical emitter configured to emit light towards a direction of the skin and at least one photodetector configured to sense the emitted light from the direction of the skin; and an array of parallel light guide elements arranged to form a plurality of parallel light paths directing light from the at least optical emitter to the at least one photodetector, each light guide element comprising, between a first end and a second end of said light guide element, an optically transparent core and an optical barrier surrounding the core, the core together with the optical barrier focusing light along the core between the ends.

## Description

### TECHNICAL FIELD

The present invention relates to a field of physiological or biometric measurements and, in particular, to optical measurements and use of an optical light guide in an optical sensor device.

### TECHNICAL BACKGROUND

A photoplethysmogram (PPG) sensor is an example of a heart activity sensor. A PPG sensor conventionally comprises at least one light source, such as a light emitting diode (LED), and at least one photodetector such as a photodiode. Light emitted by the LED(s) is directed to a skin of a user wearing the PPG sensor, and the light is delivered via the skin to the photodiode(s). For the accurate PPG measurements, it is important to deliver the light from the LED(s) to the photodiode(s) via the skin. Any light delivered directly from the LED(s) to the photodiode(s) is interference. Other optical biometric sensor may experience similar interference.

### BRIEF DESCRIPTION

The present invention is defined by the subject matter of the independent claim.

Embodiments are defined in the dependent claims.

### BRIEF DESCRIPTION OF THE DRAWINGS

In the following the invention will be described in greater detail by means of preferred embodiments with reference to the accompanying drawings, in which
Figure 1 illustrates a system to which embodiments of the invention may be applied;
Figure 2 illustrates optical biometric measurements;
Figures 3 and 4 illustrate a sensor head for optical biometric measurements according to some embodiments;
Figure 5 illustrates an embodiment of a light guide element;
Figure 6 illustrates implementation of a sensor head according to an embodiment in a wrist device;
Figures 7 and 8 illustrate a layered structure of a sensor head according to some embodiments;
Figure 9 illustrates a block diagram of a structure of a sensor device according to an embodiment;
Figures 10 and 11 illustrate some embodiments of processes for employing an active-pixel sensor array in optical biometric measurements; and
Figure 12 illustrates a process for dynamically configuring the emitters and photodetectors during optical biometric measurements.

### DETAILED DESCRIPTION OF THE INVENTION

The following embodiments are exemplifying. Although the specification may refer to "an", "one", or "some" embodiment(s) in several locations of the text, this does not necessarily mean that each reference is made to the same embodiment(s), or that a particular feature only applies to a single embodiment. Single features of different embodiments may also be combined to provide other embodiments.

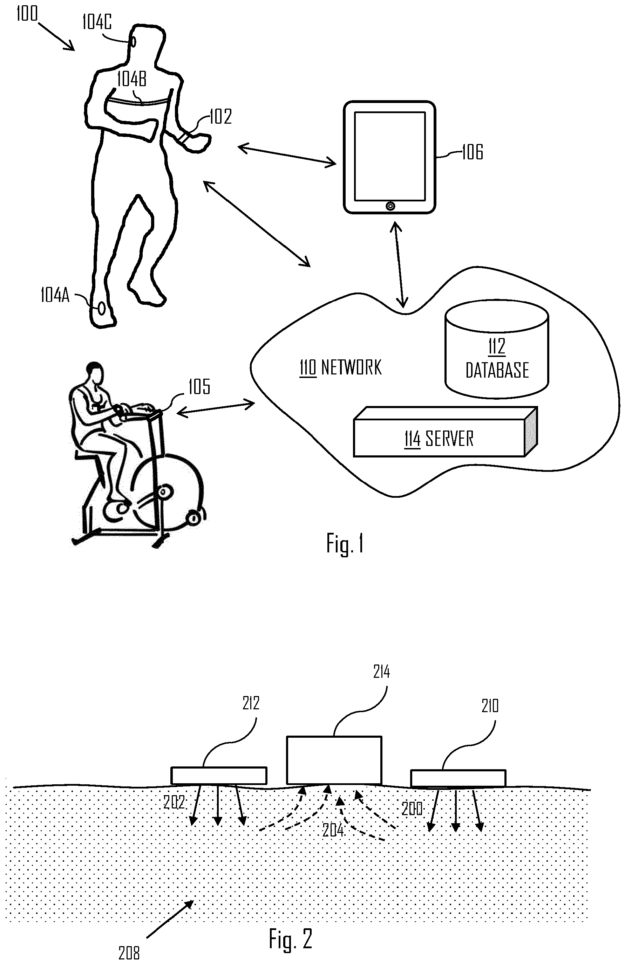

Figure 1 illustrates a system to which embodiments of the invention may be applied. Said system may comprise a training computer used to monitor physical training, activity, and/or inactivity of a user 100. The system may be configured to measure a user 100 during one or more physical exercises and/or to monitor physical activity and/or inactivity of the user 100 during the day and/or night (e.g. 24 hours a day). Such may be possible by using one or more devices described with respect to Figure 1 and in the embodiments below.

Referring to Figure 1, the user 100 may wear a wearable device, such as a wrist device 102, a head sensor unit 104C, a torso sensor 104B, and/or a leg sensor 104A. In another example, the wearable device may be and/or be comprised in glasses. In another example, the wearable device is comprised or configured to be coupled with a garment or garments (or apparel). Examples of such garments may include bra(s), swimming apparel such as swimming suit or cap, glove(s), a harness, a shirt, a band, or a vest. The garment or apparel may be worn by the user. In some embodiments, the wearable device is integrated as a part of the garment or apparel.

A typical embodiment of such a wearable device configured to measure the user is a wrist device 102. The wrist device 102 may be, for example, a smart watch, a smart device, sports watch, and/or an activity tracking apparatus (e.g. bracelet, arm band, wrist band). The wrist device 102 may be used to monitor physical activity of the user 100 by using data from internal sensor(s) comprised in the wrist device 102, data from external sensor device(s) 104A-C, and/or data from external services (e.g. training database 112). It may be possible to receive physical-activity-related information from a network 110, as the network may comprise, for example, physical activity-related information of the user 100 and/or some other user(s). Thus, the wrist device 102 may be used to monitor physical activity related information of the user 100 and/or the other user(s). The network 110 may connect the wrist device to a training database 112 and/or the server 114. The server 114 may be configured to enable data transfer between the training database 112 and some external devices, such as the wrist device and/or other wearable devices. Other examples of the wearable device include the devices illustrated in Figure 1 and described above.

The wearable device 102 may comprise an optical biometric sensor configured to measure the user. The biometric sensor may be a photoplethysmogram (PPG) sensor configured to determine cardiac activity of the user 100, such as heart rate, heart beat interval (HBI) and/or heart rate variability (HRV), for example. The PPG sensor may also (or alternatively) be used for measuring oxygen saturation (Sp02) or pulse oximetry.

Figure 2 illustrates an example of a sensor head of an optical biometric sensor, comprising multiple optical emitters such as light emitting diodes (LEDs) 210,212 and a photodetector such as a photodiode 214. The optical measurements may comprise the LEDs 210, 212 emitting light 200, 202 towards body tissue 208 of the user 100 and measuring the bounced, reflected, diffracted, scattered and/or emitted light 204 from the body tissue of the user 100 by using the photodetector 214. The emitted light is modulated when travelling through veins of the user 100 and the modulation may be detected by the optical cardiac activity sensor unit. By using detected optical measurement data, converted by the photodetector(s) into electrical measurement signals, the various characteristics of the user may be detected, such as a heart rate, oxygen saturation, blood pressure, and sleep quality.

It also needs to be noted that the sensor head may produce raw measurement data of the measured biometric characteristic and/or it may process the measurement data into biometric data, such as the heart rate. In the latter embodiments, the sensor head may comprise data processing capabilities. Also, the wrist device 102 and/or some other wearable device may comprise a processing circuitry configured to obtain the cardiac activity measurement data from the cardiac activity circuitry and to process said data into cardiac activity information, such as a cardiac activity metric characterizing the cardiac activity of the user 100. For example, the measurement data of the optical cardiac activity sensor unit may be used, by the processing circuitry, to determine heart rate, HRV and/or HBI of the user 100. Further, the raw measurement data and/or processed information may be processed by the wrist device 102 or some other wearable device, and/or transmitted to an external device, such as the portable electronic device 106.

The wrist device 102 (or more broadly, the wearable device) may comprise other types of sensor(s). Such sensor(s) may include a Laser Doppler-based blood flow sensor, a magnetic blood flow sensor, an Electromechanical Film (EMFi) pulse sensor, a temperature sensor, a pressure sensor, an electrocardiogram (ECG) sensor, and/or a polarization blood flow sensor.

Measuring cardiac activity of the user with the optical cardiac activity sensor unit (referred to simply as OHR), may be affected by motion artefacts. That is, motion artefacts may cause an effect on the measured cardiac activity signal. The effect may cause the information carried by the signal to be erroneous and/or incomplete. Some embodiments described below provide a solution to reduce the effect of motion artefacts on a cardiac activity signal measured using the OHR. The solution may enable the users to receive even more accurate cardiac activity information to help them, for example, during physical training or to plan their future training sessions.

In addition to wearable devices such as the wrist device 102 or a head sensor 104C, optical biometric measurement capability may be provided in a sensor device that is not specifically worn. For example, some training equipment such as gym devices 105 may be equipped with a sensor head capable of performing optical biometric measurements by employing the PPG, for example. Such a sensor head may be provided in a handlebar of a gym device such as a treadmill, a stationary bicycle, or a rowing machine.

Figure 2 described above illustrates the basic principle of an optical biometric sensor where the skin 208 or tissue is illuminated by one or more optical emitters 210, 212 emitting light towards the skin or tissue. The light then travels through the skin or tissue to one or more photodetectors 214 configured to detect the light transmitted by the optical emitter(s). Depending on the measurement conditions, e.g. how well the sensor head comprising the emitter(s) and the photodetector(s) is attached or positioned with respect to the skin or tissue, a certain amount of light noise travels to the photodetector(s). The light noise may comprise light from the emitter(s) that travels directly to the photodetector(s) without travelling through the skin or tissue, ambient light, etc. The light noise may induce a 'DC' bias to a measurement signal measured by the photodetector(s) that may degrade the performance of the measurements. For example, a strong DC component may saturate the photodetector(s), thus degrading the capability of detecting desired light components that have travelled through the skin.

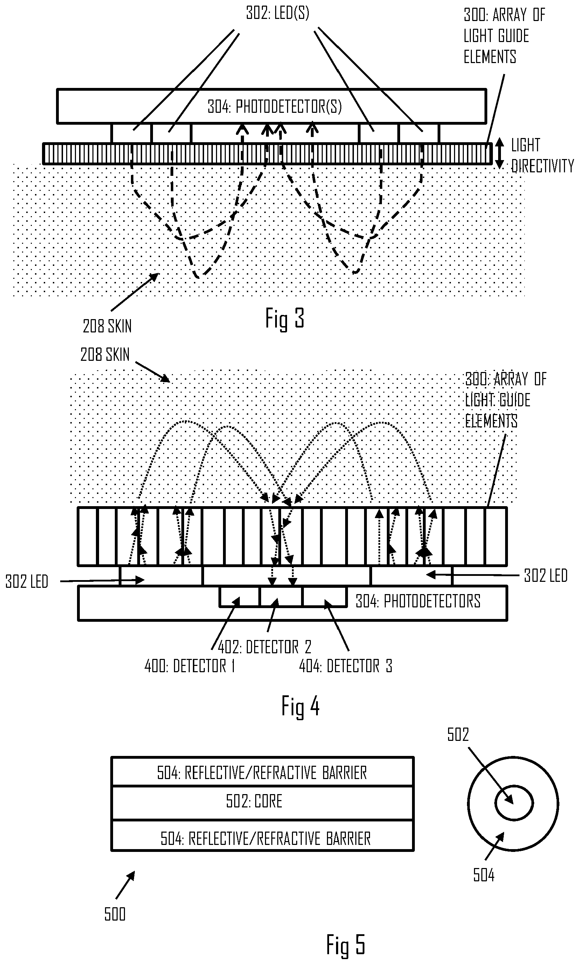

Figure 3 illustrates a sensor head of a biometric sensor device according to an embodiment. In addition to the sensor head illustrated in Figure 3, the sensor device may include an attachment mechanism for attaching the sensor device to a human body. The sensor device may be any one of the sensor devices 102, 104A to 104C illustrated in Figure 1. For example, if the sensor device is the wrist device 102 or the torso sensor 104B, the attachment mechanism may include a strap or a band attached around the user's wrist/arm or chest, respectively. In an embodiment where the sensor device is the head sensor in a form of an earpiece, the attachment mechanism may attach the sensor device to the user's ear, e.g. around the earlobe, to the concha, or inside the ear canal in the outer ear. In an embodiment where the sensor head is for the training equipment, the attachment mechanism may be omitted and the sensor head may be integrated into or attached to the gym equipment.

Referring to Figure 3, the sensor head is configured by the attachment mechanism to face the skin. The attachment mechanism may be designed such that the sensor head is directed to the skin appropriately, when attached to the human body at the appropriate location. In the training equipment, the configuration may be realized by arranging the sensor head to a location where, in use, it will naturally face and/or contact the skin, e.g. the handlebar. The sensor head comprises at least one optical emitter 302, e.g. one or more LEDs, configured to emit light towards a direction of the skin. The sensor head further comprises at least one photodetector 304 configured to sense the emitted light from the direction of the skin 208. To focus the light and reduce the light noise, an array of parallel light guide elements 300 is arranged to form a plurality of parallel light paths directing light from the at least optical emitter to the at least one photodetector. Each light guide element comprises, between a first end and a second end of said light guide element, an optically transparent core and an optical barrier surrounding the core, the core together with the optical barrier focusing light along the core between the ends.

An individual light guide element of the array may be considered to form a light pipe for the light received at one end, wherein the light is directed inside the light pipe towards the other end while preventing the light to escape the light pipe before it reaches the other end. Such a light guide element effectively focuses the light to the direction of the pipe. This is illustrated by 'light directivity' in Figure 3. Since the array is provided between the emitter(s) and the skin, light emitted by the emitter(s) 302 is first focused towards the skin by the array 300, thus effectively reducing the light scattering to other directions from the emitter(s). The closer the array is to the emitter(s), the more effective directivity towards the skin. However, the array needs not to be in direct contact with the emitter(s) to reach the desired effect. Even if an area of the array a single emitter illuminates is larger, the desired directivity towards the skin can still be achieved. The same directivity applies to the photodetector(s) 304, only to the opposite direction. Since the array is provided between the skin and the photodetector(s) 304, the light arriving at the array from the direction of the skin 208 is effectively directed towards the photodetectors by the light pipes of the array, thus effectively reducing the light from the skin scattering to other directions, thus improving the focus area of the light at a surface of the photodetector(s) 304.

The purpose of the light guide elements may thus be understood as transferring the light from one plane to another plane, wherein the planes are formed by the ends of the light pipes. Because of the array of light pipes, the transfer may be realized with low scattering of the light inside the array, thus reducing degradation of the resolution inside the array.

Figure 4 illustrates the directivity of the array in greater detail in an embodiment where there are multiple photodetectors 400, 402, 404. The photodetectors may be distributed in a plane or a surface that is parallel to the skin 208 when the sensor device is attached to the user, although Figure 4 illustrates the photodetectors directly next to one another. As illustrated in Figures 3 and 4, the light guide elements are elongated, i.e. the length of each light guide element is greater than its diameter. As described above, each light guide element effectively prevents the scattering of light to directions other than along the length of the light guide element. In other words, the light is prevented to travel from one light guide element to a neighbouring light guide element inside the light guide element. This feature is illustrated and described in greater detail in connection with Figure 5. Since the light cannot escape the light guide elements to the horizontal direction in Figure 4, the elongated light guide element effectively focuses the light travelling inside the elongated light guide element towards the direction of the length of the light guide element. In other words, the light emitted by the emitters 302 is guided upwards towards the skin by the light guide elements illuminated by the respective emitters 302, as illustrated by the arrows facing upwards in Figure 4. Then, the light penetrates the skin and propagates therein, the propagation path depending on the wavelength of the light, the alignment of the sensor head with respect to the skin, and physiological characteristics of the skin and tissues. Nevertheless, the propagation path is such that the light reflects, refracts, and scatters from the skin back towards the sensor head, as illustrated by the curved arrows reflecting from the skin back to the array in Figure 4. The light may arrive at the array at various angles, depending on the propagation of the light in the skin. Upon reaching the array from the side of the skin, the light that arrived at the various angles is again directed by the light guide element towards the vertical direction, i.e. along the length of the light guide elements. Accordingly, the direction of the light is aligned towards the detector(s) disposed facing the light guide elements that direct the reflected light. As illustrated in Figure 4, the light emitted by the emitters 302 may reach a subset of the photodetectors, depending on the conditions described above. In any case, the directivity of the array virtually brings the detectors closer to the skin, thus reducing the effect of the light noise described above. Accordingly, better signal quality can be achieved for the measurement signals output by the photodetector(s).

As illustrated in Figures 3 and 4, one end of the array is arranged to face the skin 208, and the other end the emitter(s) and the photodetector(s). Also as illustrated in Figure 3 and 4, the other end of a first subset of light guide elements of the array is arranged to face the emitter(s) and a second subset of light guide elements of the array is arranged to face the photodetector(s). Accordingly, the emitter(s) and the photodetector(s) all have 'visibility' to the skin, and the array covers the emitter(s) and the photodetector(s) such that the effect of focusing the light is achieved for both the emitter(s) and the photodetector(s).

The array may be arranged between the emitter(s) and the skin and between the photodetector(s) and the skin. The array may be in direct contact with the skin, or there may be an optically transparent layer even between the array and the skin. In the embodiments illustrated in Figures, the surface of the array that faces the skin is straight. In another embodiment, the surface facing the skin is deformed, e.g. curved to follow the contour of the skin at the attachment location. The alignment of the light guide elements may, however, be maintained such that the longitudinal axis of the light guide elements does not follow the curvature. In other words, the longitudinal direction is the same for the light guide elements in the array. In order to establish the curvature, the end of the array facing the skin may be cut or etched. As a consequence, the length of the different light guide elements may vary, according to their position in the array and the curvature.

As illustrated in Figure 4, a diameter of each light guide element may be smaller than a diameter of the photodetector(s). Furthermore, the light guide elements may be side-by-side in the array, resulting in that a plurality of light guide elements are disposed on a single photodetector to direct light to the photodetector.

In an embodiment, the array of light guide elements is formed of an array of light guide fibres. Each light guide element may thus be formed of a piece of light guide fibre, and the light guide fibres may be arranged in the form of an array having suitable dimensions to cover the emitter(s) and the photodetector(s). Figure 5 illustrates an embodiment of a structure of such a light guide element. Figure 5 illustrates a side view (on the left) and an end view (on the right) of the light guide element in Figure 5. The light guide element may comprise an optically transparent core 502 along which the light can travel freely or relatively freely from one end of the light guide element to the other end of the light guide element. An optical barrier 504 may be arranged to surround the core 502 through the length of the light guide element. The barrier or at least the surface facing the core 502 may be made of material that returns the light attempting the escape the core back towards the core via reflection or refraction. The reflective barrier may be realized by a coating of reflective material around the core. The refractive barrier may be realized by a cladding that is optically transparent or semi-transparent and has a lower refractive index than the core 502. As a consequence, the refractive barrier effectively bends the light escaping the core back towards the core. An example of a light guide element having such a refractive barrier is a graded index fibre. The array of light guide elements may thus be an array of graded index fibres.

The array of light guide elements may be arranged in a plane such that the directivity of the light guide elements is substantially perpendicular to the plane.

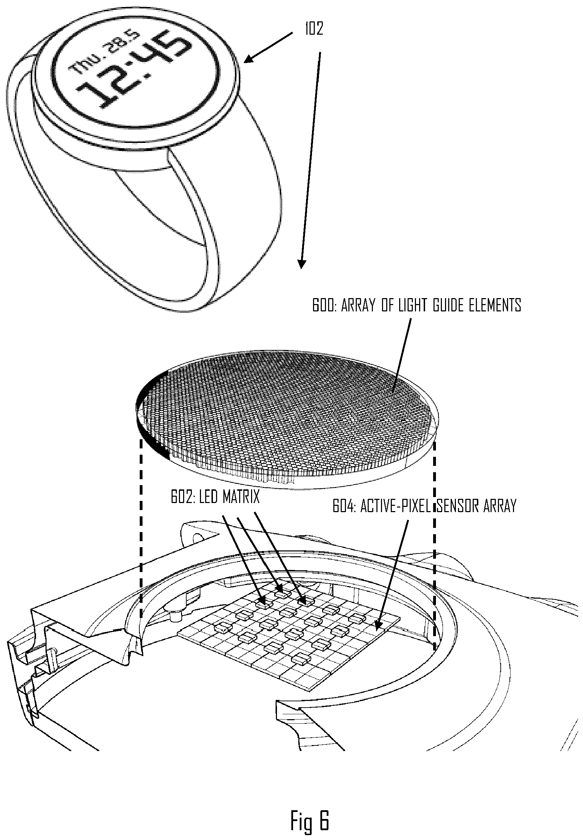

In an embodiment, the photodetector(s) form(s) an active-pixel sensor array comprising a matrix of active pixel sensors. Figures 6 to 8 illustrate such embodiments where an array of photodetectors is arranged along a plane. Figure 6 illustrates an embodiment where the sensor head is arranged in a casing of the wrist device 102. A similar sensor head may be arranged in a casing suitable for the other devices 104A to 104C, although the dimensions of the sensor head, the number of emitters and the number of photodetectors may vary. Also, the dimensions of the array of light guide elements may vary according to the number and positioning of the emitters and the photodetectors.

Referring to Figure 6, the sensor head may include a fibre optic plate 600, representing an embodiment of the array of light guide elements, an active-pixel sensor array representing an embodiment of the photodetectors, and a LED matrix 602 representing an embodiment of the optical emitters. As illustrated in Figure 6, the LED matrix 602 may be disposed on the active-pixel sensor array 604 arranged inside the casing such that the LED matrix and the active-pixel sensor array are exposed through a hole in the casing. The fibre optic plate 600 may then be disposed to cover the hole and the LED matrix and the active-pixel sensor array to direct the light in the above-described manner. As illustrated in Figure 6, the LED matrix may be disposed on the active-pixel sensor array. The LED matrix may include at least a first set of one or more LEDs configured to emit light at a first wavelength, e.g. for the purpose of the PPG measurements, and a second set of one or more LEDs configured to emit light at a second wavelength, e.g. for the purpose of motion compensation or for oxygen saturation. Each set may be arranged in the form of a matrix. For example, as illustrated in Figure 6 a two LEDs of different wavelengths may be arranged as a pair in an element of the matrix. Each pair may comprise a red LED and a green LED. The LEDs may be arranged on the active-pixel sensor array such that the LEDs cover none of the photodetectors (pixels) of the active-pixel sensor array completely. The LEDs may be arranged, for example, such that each LED covers one or more photodetectors of the active-pixel sensor array but none of them completely. Accordingly, the LEDs need not to degrade the resolution or blind any one of the photodetectors.

In an embodiment, the photodetectors of the active-pixel sensor array 604 are formed by metal-oxide semiconductor (MOS) sensors, e.g. complementary MOS (CMOS) sensors. The CMOS sensors have conventionally been used in cameras. Use of the CMOS sensors in the form of an active-pixel sensor array provides a matrix of photodetectors to capture 'a PPG image' of a resolution limited by the number of pixels in the matrix. The fibre optic plate 600 together with the active-pixel sensor array improves focusing the measurements only to the pixels reached by the desired signal (light) reflected from the skin, thus improving a signal-to-noise ratio of the measurements and reducing the light noise. When the diameter of the light guide elements is smaller than a detection area of an individual active-pixel sensor, the light delivered by the light guide element can be completely focused on the detection area, also improving the signal-to-noise ratio.

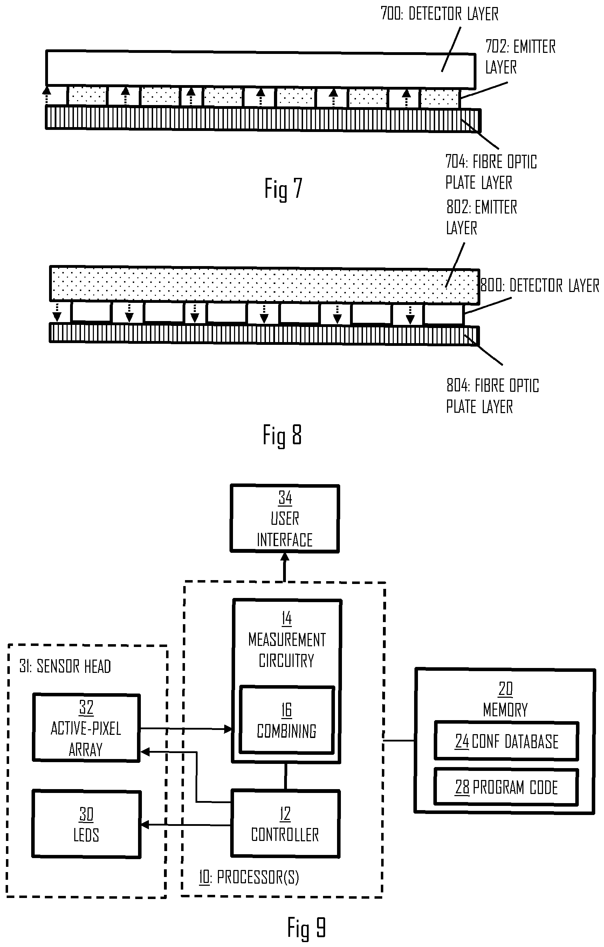

As described above and illustrated in the Figures, the emitters, the photodetectors, and the array of light guide elements may form a layered structure where the least one photodetector forms a detector layer and the array of light guide elements forms a fibre optic plate layer disposed between the detector layer and the skin. Furthermore, the at least one optical emitter forms an emitter layer, and the fibre optic plate layer is disposed between the skin and the emitter layer. Let us now describe some embodiments of the layered structure with reference to Figures 7 and 8.

In the embodiment of Figure 7, the emitter layer 702 is disposed between the fibre optic plate layer 704 and the detector layer 700, and the emitter layer 702 comprises a plurality of optical paths through the emitter layer. The optical paths may be gaps between the emitters of the emitter layer that allow delivery of light to the detector layer 700. The gaps may be air gaps or another medium transparent to the light.

In the embodiment of Figure 8, the detector layer 800 is disposed between the emitter layer 802 and the fibre optic plate layer 804 and comprises a plurality of optical paths through the detector layer 800. The optical paths may be gaps between the detectors of the emitter layer that allow delivery of light from the emitter layer to the fibre optic plate layer and ultimately to the skin. The gaps may be air gaps or another medium transparent to the light. The detector layer may be substantially transparent in an optical sense, e.g. a transparent CMOS layer.

In an embodiment, the sensor device further comprises a processing circuitry configured to receive measurement signals, responsive to the sensed light as converted by the photodetector(s), from a plurality of active-pixels sensors of the active-pixel sensor array, to process the measurement signals and to determine a physiological parameter or a biometric of the user as a result of the processing. The parameter may be a heart activity parameter such as a heart rate or a heart rate variability, or it may be an oxygen saturation parameter.

Let us then describe an embodiment of the sensor device with reference to Figure 9. The sensor device may be a wearable device comprising the attachment mechanism to attach the sensor device to the user to make it wearable. The sensor device may comprise a sensor head 31 comprising the above-described at least one optical emitter (e.g. the LEDs 30) and at least one photodetector (e.g. the active-pixel sensor array 32). The sensor device may further comprise a processing circuitry comprising at least one processor 10. The processing circuitry may comprise a controller 12 configured to control the emitter(s) 30 to emit the light according to a control sequence. As described in greater detail below, the controller may control a subset of the emitters to emit light at a time according to the control sequence. The control sequence may be fixed or it may be adaptive, as described in greater detail below. The sensor device may further comprise a measurement circuitry 14 configured to process measurement signals received from the photodetectors 32. The measurement circuitry 14 may comprise a combining circuitry 16 configured to combine at least some of the measurement signals. Some embodiments of the combining are described below.

The controller 12 may also control the photodetectors to measure a measurement signal according to the control sequence in which the emitters are activated, as described in greater detail below.

The sensor device may further comprise a communication interface providing the sensor device with wireless communication capability according to a radio communication protocol. The communication interface may support Bluetooth^{®} protocol, for example Bluetooth Low Energy or Bluetooth Smart. The communication interface may be used for configuring the sensor head or updating a computer program product configuring the operation of the sensor head. For example, the control sequence may be configured via the communication interface.

The training computer may further comprise a user interface 34 comprising a display screen, a loudspeaker, and input means such as buttons and/or a touch-sensitive display. The processor(s) 10 may output the parameter(s) computed from the measurement data to the user interface 34. On the other hand, the processor(s) 10 may receive, from the user interface 34, user input commands triggering a measurement mode. As a response to such a user input command, the controller 12 may enable or reconfigure the sensor head for the optical biometric measurements. In some embodiments, the reconfiguration may include changing a sampling rate of the optical biometric measurements, changing a set of enabled emitters and/or photodetectors, etc.

The sensor device may further comprise or have access to at least one memory 20. The memory 20 may store a computer program code 24 comprising instructions readable and executable by the processor(s) 10 and configuring the above-described operation of the processor(s). The memory 20 may further store a configuration database 28 defining parameters for the processing circuitry, e.g. the control sequence for activating the emitter(s) and/or photodetector(s).

As used in this application, the term 'circuitry' refers to all of the following: (a) hardware-only circuit implementations, such as implementations in only analog and/or digital circuitry, and (b) combinations of circuits and software (and/or firmware), such as (as applicable): (i) a combination of processor(s) or (ii) portions of processor(s)/software including digital signal processor(s), software, and memory(ies) that work together to cause an apparatus to perform various functions, and (c) circuits, such as a microprocessor(s) or a portion of a microprocessor(s), that require software or firmware for operation, even if the software or firmware is not physically present. This definition of 'circuitry' applies to all uses of this term in this application. As a further example, as used in this application, the term 'circuitry' would also cover an implementation of merely a processor (or multiple processors) or a portion of a processor and its (or their) accompanying software and/or firmware.

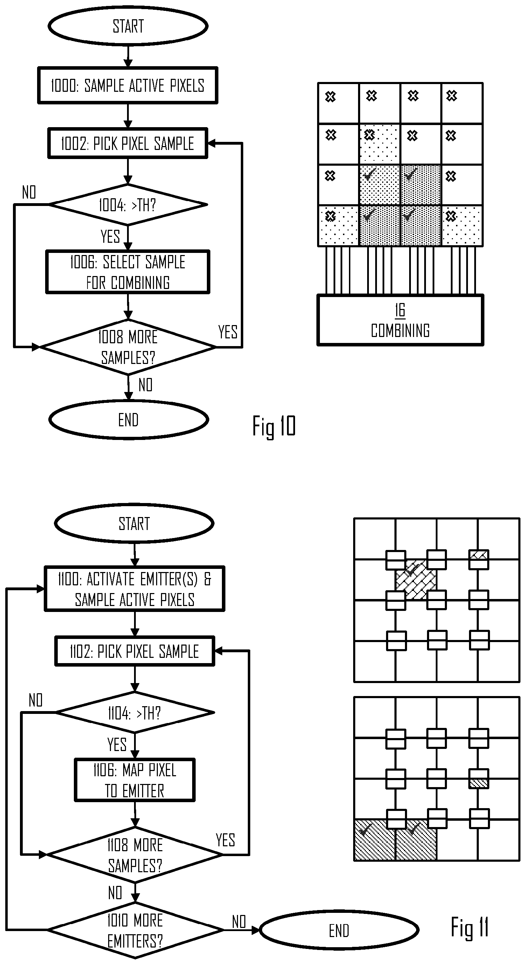

The active-pixel sensor array enables capturing a high-resolution image of the skin tissues, wherein the resolution is defined by the number of pixels in the sensor array. Such an array may enable a larger detection area than a photodiode and it also enables flexible adaptation of the detection area, as described below. Since the array of light guide elements has a 'resolution' at least as high as the resolution of the active-pixel sensor array, i.e. a single light guide element has a smaller diameter than a sensing diameter of a single pixel, the resolution of the sensor array is not degraded by the light guide elements. Instead, the light guide elements improve the focus of the light from the skin towards the respective pixel sensor(s), thus improving the signal-to-noise ratio at those pixel sensors that detect the light. These characteristics may enable embodiments described below with reference to Figures 10 and 11.

As described above, the combining circuitry may be configured to combine the received measurement signals. The combining may be based on a signal level measured at each photodetector of the active-pixel sensor array. Figure 10 illustrates a procedure for the combining circuitry according to such an embodiment. Figure 10 also illustrates a simplified active-pixel sensor array of size 4x4 pixels with signal levels illustrated by the density of a dotted pattern in each pixel. Referring to Figure 10, while the emitter(s) are enabled to emit the light to the skin, the photodetectors may be enabled by the controller to measure a light intensity in the active-pixel sensor array, and the measurements are sampled in step 1000. In step 1000 the light may be converted into electrical signals by the photodetectors and sampled into digital samples. In step 1002, a sample or a set of samples of a pixel is picked for analysis. A signal level of the sample (set) is compared with a threshold in step 1004. If the signal level is above the threshold, it is determined that the light from the emitter(s) has been detected by the photodetector forming the pixel, and the sample (set) is selected for combining (step 1006). If the signal level is below the threshold, the sample (set) may be discarded. In such a case, it may be deemed that the light emitted by the emitters did not reach the particular photodetector. If there are more samples or pixels left to be analysed in step 1008, the process may return to step 1002 for selection of the next unprocessed pixel. Otherwise, the process may end. After all the pixels have been analysed in the process of Figure 10, the samples stored for combining in step 1006 may be combined. Figure 10 illustrates the selected pixels by a tick while the pixels having the cross are excluded from the combining. As a consequence, only the pixels capable of detecting the light emitted by the emitters are taken into the computation of the physiological parameter(s) following the combining. Those pixels incapable of detecting the light emitted by the emitters may be excluded, thus reducing the light noise. Figure 10 thus illustrates an embodiment where measurement signals that exhibit a signal level above the threshold are combined and at least one measurement signal exhibiting a signal level below the threshold is excluded from the combining.

Figure 11 illustrates an embodiment for calibrating the sensor head by determining emitter-detector pairs that are able to 'communicate'. The communication may be understood in a sense that the detector is capable of detecting light emitted by the emitter. As described above, the light emitted by the emitter reflects and refracts from the skin, and the light may arrive at various locations in the active-pixel sensor array, depending on the position of the sensor head with respect to the skin, the wavelength of the emitted light, and the characteristics of the skin tissue. Increasing the sensing area naturally increases the probability of capturing all the light, but at the same time the light noise may increase. Also performing the detection with all photodetectors of the sensor array consumes power. Analogously to the emitters, there may be one or more emitters that cannot be detected by the photodetectors or by at least a determined number of photodetectors to capture sufficient measurement data.

Referring to Figure 11, the controller may enable one or more emitters to emit light to the skin, and the active-pixel sensor array to measure a light intensity in step 1100, and the measurements are also sampled in step 1100 in the above-described manner. The controller may also store the emitter(s) that emitted the light. In step 1102, a pixel sample is selected for analysis. The value of the sample may again represent a light intensity at the respective pixel when making the measurement. In step 1104, the value of the sample is compared with a threshold. The threshold may be the same as in the process of Figure 10 or a different threshold. However, the purpose of the threshold may be to determine whether or not the respective pixel has detected a sufficient light intensity of the light emitted by the emitter(s). If the value is higher than the threshold, the pixel that provided the sample may be mapped to the emitter(s) that were activated in step 1100 (step 1106). If there are more samples or pixels left to be analysed in step 1108, the process may return to step 1102 for selection of the next unprocessed pixel. Otherwise, the process may proceed to block 1010 where it is determined whether or not more emitter-detectors pairs shall be formed. In case it is determined that further pairs shall be formed, the process may return to block 1100 for selection of one or more emitters that have not yet been enabled in block 1100, and the process may proceed in the above-described manner for a different set of emitters. Otherwise, the process may end.

The controller may activate one or more emitters emitting the light at the same wavelength and/or one or more emitters emitting the light at different wavelengths. The light received in the photodetectors at different wavelengths may be discriminated by using filters and, as a consequence, the measurement signals representing the light at different wavelengths may be assigned to different processing paths. The light received at the photodetectors from the multiple emitters emitting the same wavelength may become combined readily in the skin or in the photodetector.

The procedure of Figure 11 enables calibration of the sensor head by determining adaptively the emitter-detector pairs. It enables the controller, for example, to enable only the photodetector(s) mapped to emitter(s) enabled to emit the light at a given time while disabling the other photodetector(s) not mapped to the emitter(s). Similarly, the controller may enable a determined subset of emitters that are capable of transmitting light detectable by the photodetector(s) while disabling another subset of emitters. As a consequence, power savings can be gained. It also enables forming multiple spatial measurement channels adaptively in the sensor head. For example, as illustrate in the arrays of Figure 11, one pixel indicated by the tick and the brick pattern is mapped to one emitter while other pixels indicated by the tick and the diagonal line pattern are mapped to another emitter. Since the pixels are so separated that none of the pixels is capable of detecting both emitters, two substantially orthogonal measurement channels are effective formed. This means that the emitters may emit concurrently even at the same wavelength and the two measurements may be kept separated by the knowledge of the mapping. One measurement channel is formed by a signal measured by the photodetector having the brick pattern in Figure 11, while another measurement channel is formed by signals measured by the photodetectors having the line pattern in Figure 11. The signals of the latter measurement channel may be combined according to the embodiment of Figure 10, for example. In the same manner, even further measurement channels may be adaptively formed. The sensor device may carry out recalibration to detect possible changes to the mapping, e.g. when the position of the sensor head changes. Then, new emitter-detector pairs or groups may be formed according to Figure 11.

The active-pixel sensor array and multiple emitters enables relatively free and dynamic scaling of detection capability during the optical measurements. When the measurement conditions are good, a low number of emitters and a low number of photodetectors may provide measurement signals with sufficient quality. When the measurement conditions are poor, the high number of emitters and detectors may be employed to improve the quality of the measurement signals by enabling a higher number of photodetectors (pixels) for better sensitivity and/or a higher number of emitters for better illumination of the tissue.

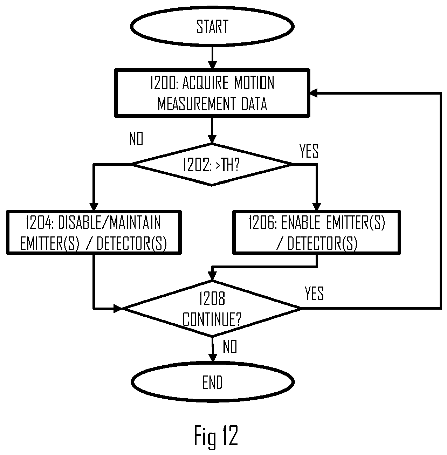

Figure 12 illustrates an embodiment where a motion sensor comprised in the sensor device is used for scaling the number of emitters and/or photodetectors enabled for the measurements. The motion sensor may comprise an accelerometer, a gyroscope, and/or a magnetometer, for example. Even a satellite positioning receiver (GPS, Galileo, Glonass,..) may be used as the motion sensor for this purpose. Figure 12 may be executed by the controller during a physical exercise performed by the user or as a part of daily monitoring of the user.

Referring to Figure 12, an initial situation may be that a certain number of emitters and a certain number of photodetectors may be currently enabled to perform the optical biometric measurements. In block 1200, motion measurement data is acquired from at least one motion sensor of the sensor device. The motion measurement data may represent a degree of motion, e.g. a degree of measured acceleration, speed, or velocity. In block 1202, the measurement data is compared with a threshold. The threshold may be preset to define a threshold where the motion is considered so high that more emitters and/or detectors are needed for sufficient measurement quality. If the measurement data indicates motion above the threshold, the process may proceed to block 1206 where one or more additional emitters and/or one or more additional photodetectors are enabled for the optical biometric measurements. When employed together with the embodiment of Figure 11, block 1206 may comprise activating an additional emitter and/or photodetector mapped to an emitter-detector pair or set currently enabled. Alternatively, or additionally, block 1206 may comprise enabling a new emitter-detector pair or set to perform the optical biometric measurements, thus enabling a new measurement channel for heart rate or oxygen saturation measurements. On the other hand, if the measurement data indicates motion below the threshold, the process may proceed to block 1204 where the current set of enabled emitter(s) and detector(s) is maintained or one or more emitters and/or one or more photodetectors of the currently-enabled set is disabled. Determining whether or not to maintain or disable may be based on a difference between the motion and the threshold. When the motion is lower than the threshold by at least a determined amount, the disabling may be chosen. When the motion is close to the threshold, the maintenance may be chosen. In block 1208, it is determined whether or not to continue the adaptive configuration of the sensor head. If the adaptive configuration continues, e.g. if the physical exercise still continues or if a significant change is detected in the motion measurement data, the process may return to block 1200 to acquire new measurement data. Otherwise, the process may end.

Instead of the motion, a signal quality of the measurement data may be estimated and compared with a signal quality threshold to determine whether or not to enable further emitter(s) and/or detector(s). In such an embodiment, block 1200 is replaced by estimation of the signal quality of the measurement data received from the currently-enabled photodetectors by the processor. If the signal quality is above the signal quality threshold, block 1204 may be executed. If the signal quality is below the threshold, block 1206 may be executed.

Yet another embodiment relates to sleep analysis. Upon detecting from the motion measurement data, heart rate, etc. that the user has fallen asleep, the controller may configure the sensor head for a sleep measurement mode. In the sleep measurement mode, the controller may change the number of enabled emitters and detectors according to a determined pattern. For example, the controller may periodically enable a higher number of emitters and detectors to make more accurate sleep analysis measurements while other times a lower number of emitters and detectors may be enabled. The controller may, for example, maintain emitter(s) and detector(s) measuring the heart rate enabled substantially for the whole duration sleep but enable emitter(s) and detector(s) measuring the oxygen saturation only intermittently.

Yet another embodiment of Figure 12 relates to the oxygen saturation measurements. Instead of the motion sensors, a barometer may be employed to enable the emitter(s) and detector(s) for measuring oxygen saturation. Block 1200 may be replaced by a block of receiving altitude measurement data from the barometer. The altitude may be compared with a threshold defining a degree of change in the measured altitude. If the change is above the threshold, indicating that the user has ascended or descended more than an amount defined by the threshold, the process may proceed to block 1206 where the oxygen saturation measurement is enabled. Otherwise, the current measurement configuration may be maintained in block 1204.

Yet another embodiment for enabling or disabling the emitter(s) and/or detector(s) is user input. For example, the user may manually trigger certain optical biometric measurements such as one-time measurement of the oxygen saturation. As another example, the user may manually control the measurement sensitivity via the user interface. If the user indicates by user input that better sensitivity is required, block 1206 may be executed.

Other embodiments may employ other criteria for scaling the number of enabled emitters and detectors to provide versatility to the optical biometric measurements.

The processes or methods described herein may be implemented by various means. For example, these techniques may be implemented in hardware (one or more devices), firmware (one or more devices), software (one or more modules or one or more computer program products), or combinations thereof. For a hardware implementation, the apparatus(es) of embodiments may be implemented within one or more application-specific integrated circuits (ASICs), digital signal processors (DSPs), digital signal processing devices (DSPDs), programmable logic devices (PLDs), field programmable gate arrays (FPGAs), graphics processing units (GPUs), processors, controllers, micro-controllers, microprocessors, other electronic units designed to perform the functions described herein, or a combination thereof. For firmware or software, the implementation can be carried out through modules of at least one chipset (e.g. procedures, functions, and so on) that perform the functions described herein. The software codes may be stored in a memory unit and executed by processors. The memory unit may be implemented within the processor or externally to the processor. In the latter case, it can be communicatively coupled to the processor via various means, as is known in the art. Additionally, the components of the systems described herein may be rearranged and/or complemented by additional components in order to facilitate the achievements of the various aspects, etc., described with regard thereto, and they are not limited to the precise configurations set forth in the given figures, as will be appreciated by one skilled in the art.

It will be obvious to a person skilled in the art that, as the technology advances, the inventive concept can be implemented in various ways. The invention and its embodiments are not limited to the examples described above but may vary within the scope of the claims.

## Claims

1. A sensor device for optical biometric measurements, comprising:
a sensor head configured to face a skin of a human body, the sensor head comprising at least one optical emitter configured to emit light towards a direction of the skin and at least one photodetector configured to sense the emitted light from the direction of the skin and to convert the sensed light into a measurement signal;
an array of parallel light guide elements arranged to form a plurality of parallel light paths directing light from the at least optical emitter to the at least one photodetector, each light guide element comprising, between a first end and a second end of said light guide element, an optically transparent core and an optical barrier surrounding the core, the core together with the optical barrier focusing light along the core between the ends; and
a processing circuitry configured to compute a biometric parameter on the basis of the measurement signal.

2. The sensor device of claim 1, wherein each light guide element is elongated along a path from the first end to the second end, and a length of each light guide element is from the first end to the second end is greater than a diameter of the light guide element.

3. The sensor device of claim 1 or 2, wherein the first end of the array of light guide elements is arranged to face the skin, and wherein the second end of a first subset of light guide elements of the array is arranged to face the at least one optical emitter and a second subset of light guide elements of the array is arranged to face the at least one photodetector.

4. The sensor device of claim 3, wherein a surface formed by the first ends of the array is curved.

5. The sensor device of any preceding claim, wherein a diameter of each light guide element is smaller than a diameter of a photodetector of the at least one photodetector, and wherein a plurality of light guide elements are disposed on the photodetector to direct light to the photodetector.

6. The sensor device of any preceding claim, wherein the array of light guide elements is formed by an array of graded index fibres.

7. The sensor device of any preceding claim, wherein the at least one photodetector forms a detector layer and the array of light guide elements forms a fibre optic plate layer disposed between the detector layer and the skin.

8. The sensor device of claim 7, wherein the at least one optical emitter forms an emitter layer disposed between the fibre optic plate layer and the detector layer, and wherein the emitter layer comprises a plurality of optical paths through the emitter layer.

9. The sensor device of claim 7, wherein the at least one optical emitter forms an emitter layer, and wherein the detector layer is disposed between the emitter layer and the fibre optic plate layer and comprises a plurality of optical paths through the emitter layer.

10. The sensor device of any preceding claim, wherein the at least one photodetector forms an active-pixel sensor layer comprising a matrix of active pixel sensors.

11. The sensor device of claim 10, wherein the processing circuitry is configured to receive measurement signals, responsive to the sensed light, from a plurality of active-pixels sensors of the active-pixel sensor layer, and to combine the measurement signals of the plurality of active-pixels sensors.

12. The sensor device of claim 11, wherein the processing circuitry is configured to combine the measurement signals that exhibit a signal level above a threshold and to exclude from the combining at least one measurement signal exhibiting a signal level below the threshold.

13. The sensor device of any preceding claim 10 to 12, wherein the processing circuitry is configured to dynamically enable and disable the plurality of active-pixel sensors during measurements.

14. The sensor device of any preceding claim, wherein the sensor head is comprised in at least one of an optical heart activity sensor, an oxygen saturation sensor.

15. The sensor device of any preceding claim, further comprising an attachment mechanism for attaching the sensor device to the human body.
